# EUROPEAN PATENT APPLICATION

(11) **EP 1 129 680 A1**
(43) Date of publication of application: **05.09.2001**
(21) Application number: 01301796.7
(22) Date of filing: 27.02.2001
(51) Int. Cl.: A61F 5/445

(54) **Ostomy bags**

(30) Priority: 04.03.2000 GB 0005129; 19.07.2000 GB 0017596
(71) Applicant: Smiths Group plc, London, NW11 8DS (GB)
(72) Inventor: Whiteside, Neil Adrian, Steyning, Sussex BN44 3NE (GB)
(74) Representative: Flint, Jonathan McNeill

(57) **Abstract**

An alkali-disposable ostomy bag has a pouch 1 with flange 2 for attachment to the patient. The flange 2 is formed from two discs one of which 22 is relatively thin and alkali disposable and is permanently attached to the pouch. The other disc 21 is thicker, with a hydrocolloid surface 24, and is attached with the disc 22 on the pouch by a peel-off adhesive 26. After use, the bag is removed from the patient and the thicker disc 21 is peeled off the thinner disc 22 so that it can be disposed of. The adhesive 26 remains on the thinner disc 22 so that the pouch 1 can be sealed and disposed of by adding an alkali to a wc pan.

## Description

This invention relates to ostomy bags of the kind comprising a flexible pouch and a flange attached with the bag by which the bag is attached to and removed from the patient, the pouch having a forward wall and a rear wall facing the patient, the rear wall having an opening therein through which waste material from the patient can enter the pouch and the flange having an opening therethrough in alignment with the opening in the rear wall.

Attempts have been made recently to develop ostomy bags which can be disposed of by flushing in a wc, to avoid the need to make special disposal arrangements, which can be inconvenient, embarrassing and unhygenic.

WC-disposable bags have been proposed in the literature, the bags having an outer water-soluble or dispersible layer and an inner water-resistant layer. The outer layer provides mechanical support for the inner layer so that, when the bag is dropped into turbulent water in a wc pan, the outer layer is quickly broken up. The inner layer prevents the contents of the bag attacking the outer layer in use but, once the outer layer is broken up on disposal, the inner layer does not have sufficient mechanical strength in itself to cause blockage on flushing the wc. An example of such a bag is described in GB 2083762B. A wc-disposable bag is sold by SIMS Portex Limited, England under the name Symphony (Symphony is a Registered Trade Mark of SIMS Portex Limited).

Although such bags can be used satisfactorily, the user has to take special precautions to ensure that the outside of the bag does not become wet, because the outer layer would be damaged by contact with water. This can be especially inconvenient with bags which are worn long-term, for two or more days, such as is usually the case with ileostomy bags. The use of such bags can make washing difficult and prevents the user swimming.

An alternative form of bag is described in EP 0142950A, which is made of 3-hydroxybutyrate film, either in a laminate with a water-soluble film as an outer layer, or entirely from 3-hydroxybutyrate. Such a material remains intact when in contact with water or body waste, but is broken up if the pH is raised to about 12. The bag described is disposed of by adding a base material to the contents of the bag so as to raise the pH of the contents to at least 12 so that it breaks up when agitated in a wc pan.

A further alkali-disposable bag is described in GB 2195919B. The walls of this bag have a central layer of polyvinyl alcohol, an inner layer of a blend of polyvinylidene chloride acrylonitrile copolymer with carboxylated acrylic copolymer, and an outer layer of two or more coatings of carboxylated acrylic acid. This bag can be disposed of in a wc by adding an alkali to the water in the pan.

Another alkali-disposable bag is described in GB 2257056B. This bag has an outer layer substantially entirely of alkali-soluble/water-insoluble carboxylated acrylic polymer forming a major part of the thickness of the material and a thinner, inner layer of alkaliresistant polyvinylidene chloride bonded directly to one side of the first layer. GB 2324761A describes an alkali-disposable bag with a non-woven outer layer.

Previous bags have a flange around their inlet opening supporting an adhesive material by which the bag is attached to the skin of the patient. In order to provide the desired security of attachment, the flange is thicker and stiffer than the walls of the bag. Although the flange can be made such that it can be flushed away in a wc, it can take longer to flush away, and the perceived bulk of the flange can make users think that it will not flush away, or that it will create a blockage.

It is an object of the present invention to provide an alternative ostomy bag.

According to one aspect of the present invention there is provided an ostomy bag of the above-specified kind, characterised in that the flange has an adhesive rear surface for attachment to the patient, and that at least a major part of the flange is removably attached with the pouch such that it can be separated from the pouch after use and disposed of separately from the pouch.

The major part of the flange is preferably attached with the bag by means of an adhesive. The major part of the flange is preferably provided by a first disc, the flange also having a second disc, the second disc being attached permanently with the rear wall of the pouch around the opening, the first disc being removably attached on its forward surface with the second disc, and the adhesive rear surface for attachment to the patient being provided on the rear surface of the first disc. The first disc is preferably attached with the second disc by an adhesive, the adhesive remaining on the second disc after removal of the first disc. The pouch is preferably wc disposable and may be alkali disposable. The second disc may be alkali disposable. The flange is preferably attached with the pouch around the opening of the flange and is preferably unattached around its outer edge.

According to another aspect of the present invention there is provided a method of collecting and disposing of body waste material, comprising the steps of securing a bag by means of an adhesive flange to the patient around a body waste opening, allowing body waste material to enter the bag, peeling the flange off the patient so as to remove the bag, removing at least a major part of the flange from the bag, disposing of the removed part of the flange, placing the remaining part of the bag. and its contents in the pan of a wc and flushing it away.

The major part of the flange may be removed from the bag by separating an adhesive bond.

An ostomy bag and its method of use, according to the present invention will now be described, by way of example, with reference to the accompanying drawings, in which:
- Figure 1: is a view of the rear side of the bag;
- Figure 2: is a sectional side elevation view of an upper part of the bag along the line II-II of Figure 1 to an enlarged scale; and
- Figures 3 to 5: illustrate steps in use of the bag.

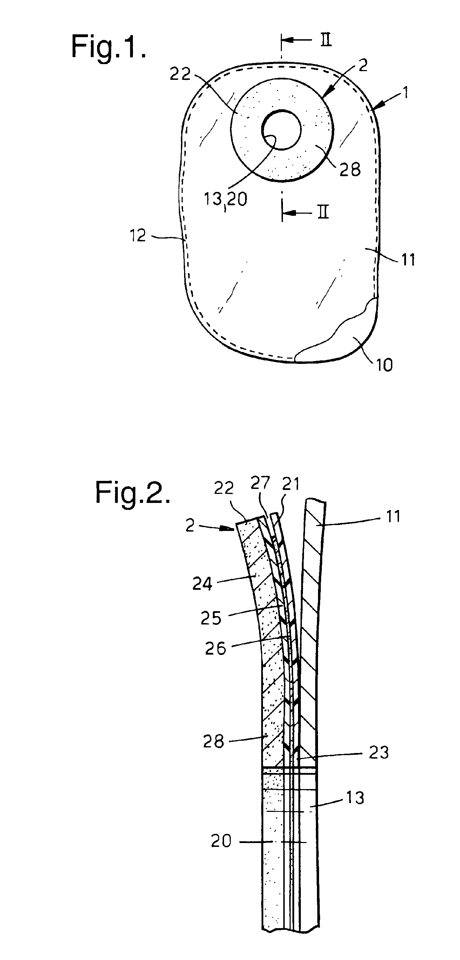

With reference first to Figures 1 and 2, the bag comprises a pouch 1 and a flange 2, a part of which is separable from the pouch after use.

The pouch 1 has a forward wall 10, facing away from the patient, and a rear wall 11, facing towards the patient and joined with the forward wall around its edge by a peripheral weld 12. The walls 10 and 11 are formed of a conventional alkali-disposable material, such as described in GB 2324761. Centrally towards its upper end, the pouch 1 has a circular opening 13 formed in the rear wall 11 through which waste material from the patient can enter the pouch. The pouch 1 may include other conventional features, such as a filter, but is otherwise made entirely of a flexible wc-disposable material.

The flange 2 has a central aperture 20 aligned with the opening 13 in the rear wall 11 and comprises two discs 21 and 22. The first, forward disc 21 is made of a thin, alkali-disposable material such as carboxylated acrylic polymer and is attached to the rear wall 11 by means of a weld 23 around the opening 13 but is otherwise unattached with the pouch 1. This enables the outer edge of the forward disc 21 to float relative to the pouch 1 so that force between the pouch and the flange is concentrated at the weld joint 23 rather than at the attachment between the two discs. Alternatively, a separate ring (not shown) could be secured to the inner surface of the wall 11, the disc 21 being attached to this where it overlaps the opening 13. The second, rear disc 22 is relatively thick, having a layer 24 of a skin-compatible, pressure-sensitive adhesive material, such as a hydrocolloid, and a thinner, flexible, non-adhesive backing sheet 25 on the forward surface of the layer 24. The rear surface of the adhesive layer 24, that is, the surface facing the patient, is protected, before use, by a removable release sheet (not shown), in the conventional way. The rear disc 22 is typically about 2mm thick, making the flange 2 stiffer than the walls of the pouch 1 but it is readily bendable to conform to the anatomy of the patient. The relative thickness of the discs and layers are not shown to scale in Figure 2.

The forward face of the rear disc 22 is attached with the rear surface of the forward disc 21 by a thin layer 26 of an adhesive. The adhesive 26 extends over the major part of the rear surface of the disc 21 apart from a peripheral margin 27 where the two discs are unattached. The nature of the adhesive 26 and the materials of the forward disc 21 and the backing sheet 25 are such that the adhesive bonds more securely with the forward disc than with the backing sheet and the bond is strong in the shear mode but is less strong in the peel mode. Alternatively, the forward disc 21 could be joined with the rear disc 22 by a rupturable weld and the forward disc could have a separate adhesive patch to enable the pouch 1 to be sealed closed after use.

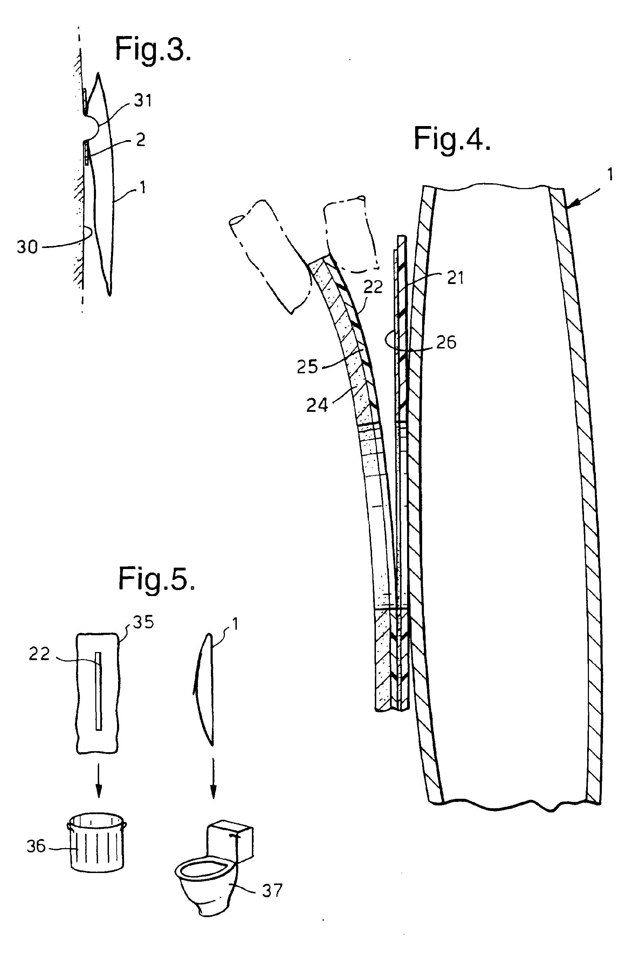

In use, the user removes the release sheet and presses the flange 2 into position on the skin 30 around the stoma 31 or other body waste opening, as shown in Figure 3. Waste material from the stoma 31 enters the bag via the opening 13 in the rear wall 11. The flange 2 provides a secure retention of the bag on the patient during normal use. When the bag is to be removed, the flange 2 is peeled away from the skin 30, in the usual way, to remove both the pouch and the flange. The user then grips the outer edges of the two discs 21 and 22 at the margin 27 and peels the thicker adhesive disc 22 away from the other disc 21 and hence off the bag, as shown in Figure 4. The adhesive 26 is selected to allow the rear disc 22 to be removed from the forward disc 21 by peeling off in this way. The user then wraps the disc 22 in a covering 35 and disposes of it after a single use in the conventional way in a waste container 36, as shown in Figure 5. The user squeezes the pouch 1 to expel excess air and folds the bag about a diameter of the disc 21 so that the adhesive 26 on the two halves of the disc contact and seal the bag closed. The pouch 1 with the forward disc 21 attached is dropped into the pan of a wc 37 into which an alkali has been added, as shown in Figure 5. The pouch 1 is quickly broken up by the action of the alkali enabling it to be flushed away. Because the disc 21 is thin and also of an alkali-disposable material, this can also be flushed away easily.

The user applies a fresh bag in the same way.

The present invention enables the major part of the flange of a bag to be removed before disposal in a wc. The invention could be modified in various ways. For example, the adhesive bond between the two discs could be provided by means of an adhesive on the front surface of the rear disc rather than on the rear surface of the front disc, although this arrangement does not allow the pouch to be sealed after use. The flange could be a single disc and attached with the pouch by means of an adhesive directly on the rear wall of the pouch. It may not be essential for the forward disc to be of a material that is broken up by an alkali if it is made thin enough so that it does not present any obstruction to flushing. The invention is not confined to use with alkali-disposable bags but could be used with other wc-disposable bags.

## Claims

1. An ostomy bag comprising a flexible pouch (1) and a flange (2) attached with the bag by which the bag is attached to and removed from the patient, the pouch having a forward wall (10) and a rear wall (11) facing the patient, the rear wall (11) having an opening (13) therein through which waste material from the patient can enter the pouch, the flange (2) having an opening (20) therethrough in alignment with the opening (13) in the rear wall (11), **characterised in that** the flange (2) has an adhesive rear surface (28) for attachment to the patient, and that at least a major part (22) of the flange is removably attached with the pouch (1) such that it can be separated from the pouch after use and disposed of separately from the pouch.

2. A bag according to Claim 1, **characterised in that** the major part (22) of the flange (2) is attached with the bag by means of an adhesive (26).

3. A bag according to Claim 1 or 2, **characterised in that** the major part of the flange is provided by a first disc (22), that the flange (2) also has a second disc (21), that the second disc (21) is attached permanently with the rear wall (11) of the pouch (1) around the opening (13), that the first disc (22) is removably attached on its forward surface with the second disc (21), and that the adhesive surface (28) for attachment to the patient is provided on the rear surface of the first disc (22).

4. A bag according to Claim 3, **characterised in that** the first disc (22) is attached with the second disc (21) by an adhesive (26), and that the adhesive (26) remains on the second disc (21) after removal of the first disc (22).

5. A bag according to any one of the preceding claims, **characterised in that** the pouch (1) is wc disposable.

6. A bag according to any one of the preceding claims, **characterised in that** the pouch (1) is disposable on contact with an alkali.

7. A bag according to Claim 6 and Claim 3 or 4, **characterised in that** the second disc (21) is alkali disposable.

8. An ostomy bag according to any one of the claims, **characterised in that** the flange (2) is attached with the pouch (1) around the opening (20) of the flange and is unattached around its outer edge.

9. A method of collecting and disposing of body waste material, comprising the steps of securing a bag by means of an adhesive flange (2) to the patient around a body waste opening (31), allowing body waste material to enter the bag, peeling the flange off the patient so as to remove the bag, removing at least a major part (22) of the flange (2) from the bag, disposing of the removed part (22) of the flange, placing the remaining part of the bag (1 and 21) and its contents in the pan of a wc (37) and flushing it away.

10. A method according to Claim 9, **characterised in that** the major part (22) of the flange (2) is removed from the bag by separating an adhesive bond (26).
